# FASCICULE DE BREVET EUROPEEN

(11) **EP 2 932 265 B1**
(45) Date de publication et mention de la délivrance du brevet: **15.08.2018**
(21) Numéro de dépôt: 13820807.9
(22) Date de dépôt: 12.12.2013
(51) Int. Cl.: G01N 33/50, G01N 33/569

(54) **SOUCHES PROBIOTIQUES POUR LE TRAITEMENT ET/OU LA PRÉVENTION DE LA DIARRHÉE**
PROBIOTISCHE STÄMME ZUR BEHANDLUNG UND/ODER VORBEUGUNG VON DURCHFALL
PROBIOTIC STRAINS FOR THE TREATMENT AND/OR PREVENTION OF DIARRHEA

(30) Priorité: 12.12.2012 FR 1261916
(43) Date de publication de la demande: 21.10.2015
(73) Titulaire: Lesaffre et Compagnie, 75001 Paris (FR); Institut National de la Recherche Agronomique (INRA), 75338 Paris Cedex 07 (FR); École Nationale Supérieure des Sciences Agronomiques de Bordeaux-Aquitaine (Bordeaux Sciences Agro), 33170 Gradignan (FR)
(72) Inventeur: KHUONG HUU, Marie, 64100 Bayonne (FR); FIORAMONTI, Jean, 31120 Roquette (FR); URDACI, Maria, 33175 Gradignan Cédex (FR)
(74) Mandataire: Cabinet Plasseraud
(86) Numéro de dépôt international: PCT/FR2013/053044
(87) Numéro de publication internationale: WO 2014/091160

(56) Documents cités:
- WO-A2-2007/064741
- G. RAHEJA ET AL: "Lactobacillus acidophilus stimulates the expression of SLC26A3 via a transcriptional mechanism", AMERICAN JOURNAL OF PHYSIOLOGY: GASTROINTESTINAL AND LIVER PHYSIOLOGY, vol. 298, no. 3, 1 mars 2010 (2010-03-01), pages G395-G401, XP055066166, ISSN: 0193-1857, DOI: 10.1152/ajpgi.00465.2009
- ALAN BR THOMSON: "Recent advances in small bowel diseases: Part I", WORLD JOURNAL OF GASTROENTEROLOGY, vol. 18, no. 26, 1 janvier 2012 (2012-01-01), page 3336, XP055066038, ISSN: 1007-9327, DOI: 10.3748/wjg.v18.i26.3336
- E. HEUVELIN ET AL: "A Bifidobacterium Probiotic Strain and Its Soluble Factors Alleviate Chloride Secretion by Human Intestinal Epithelial Cells", THE JOURNAL OF NUTRITION, vol. 140, no. 1, 1 janvier 2010 (2010-01-01), pages 7-11, XP055066032, ISSN: 0022-3166, DOI: 10.3945/jn.109.114553
- RESTA-LENERT ET AL: "Probiotics and Commensals Reverse TNF-alpha- and IFN-gamma-Induced Dysfunction in Human Intestinal Epithelial Cells", GASTROENTEROLOGY, ELSEVIER, PHILADELPHIA, PA, vol. 130, no. 3, 1 mars 2006 (2006-03-01), pages 731-746, XP005442375, ISSN: 0016-5085, DOI: 10.1053/J.GASTRO.2005.12.015
- MACFARLANE G T ET AL: "PROBIOTICS, INFECTION AND IMMUNITY", CURRENT OPINION ON INFECTIOUS DISEASES, CURRENT SCIENCES, GB, vol. 15, no. 5, 1 janvier 2002 (2002-01-01), pages 501-506, XP009081317, ISSN: 0951-7375
- KIELA PAWEL R ET AL: "Molecular mechanism of rat NHE3 gene promoter regulation by sodium butyrate", AMERICAN JOURNAL OF PHYSIOLOGY - CELL PHYSIOLOGY, vol. 293, no. 1, juillet 2007 (2007-07), pages C64-C74, XP002698832, ISSN: 0363-6143

## Description

### Domaine de l'Invention

La présente invention concerne le domaine des souches probiotiques, en particulier des souches probiotiques de *Bacillus,* utiles dans le traitement et/ou la prévention de la diarrhée. L'invention porte également sur un procédé de sélection de souches probiotiques qui ont la particularité d'agir sur l'absorption d'eau au niveau du côlon.

### Contexte de l'Invention

En fonctionnement normal, le côlon chez l'homme absorbe environ 99% de l'eau entrant dans sa lumière, ce qui représente environ 2 litres d'eau par jour. Le côlon a la capacité d'absorber jusqu'à 4 litres d'eau supplémentaires. Toutefois, au-delà de 6 litres d'eau par jour, ses capacités d'absorption sont saturées et la diarrhée apparait.

La diarrhée est un problème fréquent (environ deux milliards de cas chaque année dans le monde) qui se caractérise par des selles de consistance liquide ou molle, plus volumineuses et nombreuses qu'à l'habitude (plus de 3 selles par jour). Dans les cas extrêmes, plus de 20 litres de fluide peuvent être perdus par jour.

La diarrhée n'est pas une maladie, mais un symptôme. Sa cause la plus fréquente est l'ingestion d'eau ou d'aliments contaminés ; elle dure alors un ou deux jours sans nécessiter de traitement. Mais la diarrhée elle-même peut provoquer une déshydratation qui peut être mortelle, surtout chez le nourrisson où une perte de poids supérieure à 10% est une urgence hospitalière. D'après l'Organisation Mondiale de la Santé, les diarrhées sont la seconde cause de mortalité infantile dans les pays du tiers monde, et sont responsables de 18% des morts d'enfants de moins de 5 ans (Bryce et al., Lancet, 2005, 365: 1147-1152).

La déshydratation provoquée par la diarrhée intervient quand les pertes de fluide ne sont pas compensées. En situation normale, c'est au niveau du côlon que les selles sont asséchées. Les phénomènes de réabsorption de l'eau contenue dans les matières ingérées se produisent au niveau des cellules coliques par une combinaison de transports actifs et passifs d'eau et d'électrolytes. Au niveau des cryptes des invaginations de l'épithélium colique, une sécrétion d'eau allant du sang vers le milieu extérieur a lieu. Ces deux phénomènes se compensent chez la personne en bonne santé et permettent de maintenir une hydratation convenable des selles, ce qui favorise le transit intestinal et améliore les conditions de circulation des molécules.

Il existe de nombreuses causes possibles de diarrhées, parmi lesquelles les diarrhées d'origine infectieuse induites par un pathogène viral, bactérien ou parasitaire et les diarrhées non infectieuses, telles que les diarrhées induites par une intolérance alimentaire, un régime gras, l'alcool, un facteur psychologique, l'administration d'un médicament, l'administration d'une procédure thérapeutique, les diarrhées associées à une maladie ou à une condition clinique, ou les diarrhées associées à un sevrage de stupéfiant.

Selon la durée des symptômes, on distingue les diarrhées aiguës et les diarrhées chroniques dont les symptômes durent respectivement moins de deux semaines et au moins deux semaines.

Un médicament classique pour le traitement de la diarrhée est par exemple le lopéramide, qui agit à la fois en stimulant l'absorption d'eau et d'électrolytes et en ralentissant la durée du transit. Toutefois, le ralentissement de la durée du transit peut être problématique lors d'infections sévères du genre *Salmonella, Shigella* ou *Clostridium difficile,* puisque la durée de séjour dans l'intestin de la bactérie pathogène est prolongée.

Les probiotiques constituent une alternative intéressante dans le traitement et/ou la prévention de la diarrhée. Les probiotiques les plus utilisés pour cette indication sont les bactéries lactiques. Les probiotiques sont alors généralement décrits comme ayant une action bénéfique sur le système immunitaire et sur l'équilibre de la flore intestinale.

D'autres études ont cherché à mettre en évidence de nouvelles cibles pour le traitement ou la prévention de la diarrhée. Ainsi, le document WO 2004/028480 décrit des composés de type thiazolidinone permettant de diminuer l'efficacité du transport d'ions chlorure par la protéine CFTR (*Cystic Fibrosis Transmembrane Conductance Regulator*) et leur utilisation dans le traitement de la diarrhée sécrétoire. Bradford et al. (Am. J. Physiol. Gastrointes. Liver Physiol., 2009, 296: G886-G898) s'intéressent à des moyens de traiter les problèmes d'obstruction intestinale et de déshydratation chez des patients atteints de mucoviscidose (dont l'expression de CFTR est altérée).

Par ailleurs, il est décrit que la perte partielle ou complète de la protéine NHE3 (*Sodium Hydrogen Exchanger 3*) chez des souris atteintes de mucoviscidose réduit l'incidence des obstructions intestinales, notamment en augmentant la fluidité intestinale, et concluent que NHE3 est une cible potentielle pour réguler la fluidité du tractus intestinal chez des patients atteints de mucoviscidose et dont l'expression de CFTR est altérée. Geetu Raheja et al. (Amer. J. Physiol. Gastroint. and Liver Physiol., 2010, 298(3): G395-G401) ont montré qu'une souche probiotique induisant l'augmentation de l'expression d'un transporteur (SCL26A3) impliqué dans l'absorption de l'eau au niveau du côlon peut être bénéfique pour le traitement de la diarrhée.

Il existe donc toujours un besoin de disposer de nouvelles stratégies pour le traitement et/ou la prévention des diarrhées.

### Résumé de l'Invention

D'une façon générale, la présente invention repose sur la mise en évidence que, de manière tout à fait originale, certaines souches probiotiques sont capables d'agir directement sur la diarrhée, en induisant une absorption de l'eau présente en excès au niveau du côlon. De manière surprenante et inattendue, les Inventeurs ont montré que certaines souches probiotiques sont capables d'inhiber l'expression de transporteurs impliqués dans la sécrétion d'eau au niveau du côlon et/ou de stimuler l'expression de transporteurs impliqués dans l'absorption d'eau au niveau du côlon, et peuvent être utilisées pour le traitement et/ou la prévention de la diarrhée.

Ainsi, de manière tout à fait originale, ces souches probiotiques n'agissent pas comme des inhibiteurs des transporteurs, c'est-à-dire n'agissent pas en se fixant sur les transporteurs, entravant ainsi leur fonctionnement, mais agissent sur leur expression et donc sur le nombre de transporteurs présents.

Par ailleurs, de telles souches probiotiques agissant directement sur l'absorption de l'eau présente en excès au niveau du côlon présentent l'avantage de pouvoir être utilisées pour la prévention et/ou le traitement de tout type de diarrhée, en particulier que la diarrhée soit d'origine infectieuse ou non infectieuse et en présence ou non d'une dysbiose.

La présente invention a ainsi pour objet un procédé de sélection d'une souche probiotique pour le traitement et/ou la prévention de la diarrhée, le procédé comprenant les étapes de :
- mesure de l'effet d'au moins une souche à tester sur l'expression de la protéine CFTR (*Cystic Fibrosis Transmembrane Conductance Regulator*) dans un échantillon biologique, et
- sélection d'au moins une souche qui induit une diminution de l'expression de la protéine CFTR,
où l'échantillon biologique est un échantillon d'intestin proximal ou des colonocytes.

Dans certains modes de réalisation, le procédé de sélection d'une souche probiotique pour le traitement et/ou la prévention de la diarrhée selon l'invention, comprend en outre les étapes de :
- mesure de l'effet d'au moins une souche à tester sur l'expression de la protéine NHE3 (*Sodium Hydrogen Exchanger 3*), dans un échantillon biologique, et
- sélection d'au moins une souche qui induit une augmentation de l'expression de la protéine NHE3,
où l'échantillon biologique est un échantillon d'intestin proximal ou des colonocytes.

Dans certains modes de réalisation, la souche à tester est une souche de levure ou une souche de bactérie. En particulier, une souche de bactérie à tester peut être une souche de *Bacillus,* de préférence une souche de *Bacillus subtilis.*

Dans certains modes de réalisation, l'expression de la protéine CFTR et/ou de la protéine NHE3 est mesurée par électrophorèse, Western-blot, immunoessai, immunohistochimie, immunocytochimie, spectrométrie de masse, RT-PCR ou Northern-blot.

Une souche probiotique sélectionnée par un procédé selon l'invention peut être utilisée dans le traitement et/ou la prévention de la diarrhée.

Dans un autre aspect, la présente invention concerne une souche probiotique, ou une composition comprenant la souche probiotique, pour utilisation dans le traitement et/ou la prévention de la diarrhée, où la souche probiotique est la souche de *Bacillus subtilis* CU1 déposée à la CNCM (Collection Nationale de Cultures de Microorganismes, 25 rue du Docteur Roux, 75724 Paris Cedex 15) le 25 octobre 2001 sous le numéro I-2745.

Le présent document décrit aussi des cellules obtenues par culture d'une souche probiotique sélectionnée par un procédé selon l'invention, pour leur utilisation dans le traitement et/ou la prévention de la diarrhée, en particulier, des cellules de *Bacillus subtilis* obtenues par culture de la souche de *Bacillus subtilis* CU1 déposée à la CNCM le 25 octobre 2001 sous le numéro I-2745, pour leur utilisation dans le traitement et/ou la prévention de la diarrhée.

Le présent document décrit également une méthode de traitement et/ou de prévention de la diarrhée chez un patient comprenant l'administration au patient d'une quantité efficace de cellules obtenues par culture d'une souche probiotique sélectionnée par un procédé selon l'invention.

La mise en oeuvre de la méthode de traitement et/ou de prévention de la diarrhée comprend préférablement l'administration de cellules de *Bacillus subtilis* obtenues par culture d'une souche probiotique de *Bacillus subtilis,* de préférence la souche de *Bacillus subtilis* CU1 déposée à la CNCM le 25 octobre 2001 sous le numéro I-2745.

Les diarrhées qui peuvent être traitées de façon préventive et/ou curative selon l'invention incluent les diarrhées aiguës et les diarrhées chroniques.

Dans certains modes de réalisation, la diarrhée traitée selon l'invention est une diarrhée d'origine infectieuse qui est induite par un pathogène viral, bactérien ou parasitaire.

Dans d'autres modes de réalisation, la diarrhée traitée selon l'invention est une diarrhée d'origine non infectieuse.

Une diarrhée d'origine non infectieuse est par exemple une diarrhée induite par une intolérance alimentaire, une diarrhée induite par un régime gras, une diarrhée induite par l'alcool, une diarrhée induite par un facteur psychologique, une diarrhée induite par administration d'un médicament, une diarrhée induite par administration d'une procédure thérapeutique, une diarrhée associée à une maladie ou à une condition clinique, ou une diarrhée associée à un sevrage de stupéfiant.

Une description plus détaillée de certains modes de réalisation préférés de l'invention est donnée ci-dessous.

### Description Détaillée de l'Invention

Comme mentionné plus haut, la présente invention concerne l'identification de souches probiotiques capables d'agir sur l'absorption d'eau au niveau du côlon par action directe au niveau des transporteurs impliqués dans la régulation du fluide intestinal, et leur utilisation comme médicaments dans le traitement et/ou la prévention de la diarrhée.

### I - Procédé de Sélection de Souches Probiotiques

Un procédé de sélection décrit ici a pour but d'identifier une souche probiotique qui est utile dans le traitement et/ou la prévention de la diarrhée.

Le procédé de sélection d'une souche probiotique pour le traitement et/ou la prévention de la diarrhée comprend les étapes de :
- mesure de l'effet d'au moins une souche à tester sur l'expression d'au moins un transporteur impliqué dans l'absorption ou la sécrétion d'eau au niveau du côlon, et
- sélection d'au moins une souche qui induit une diminution de l'expression d'au moins un transporteur impliqué dans la sécrétion d'eau au niveau du côlon et/ou une augmentation de l'expression d'au moins un transporteur impliqué dans l'absorption d'eau au niveau du côlon.

Un exemple de transporteur impliqué dans la sécrétion d'eau au niveau du côlon est la protéine CFTR (*Cystic Fibrosis Transmembrane Conductance Regulator*).

Un exemple de transporteur impliqué dans l'absorption d'eau au niveau du côlon est la protéine NHE3 (*Sodium Hydrogen Exchanger 3*).

Un procédé de sélection selon l'invention est caractérisé en ce qu'il comprend :
- la mesure de l'effet d'au moins une souche à tester sur l'expression de la protéine CFTR et/ou de la protéine NHE3, et
- la sélection d'au moins une souche qui induit une diminution de l'expression de la protéine CFTR,
et optionnellement :
- la mesure de l'effet d'au moins une souche à tester sur l'expression de la protéine NHE3, et
- la sélection d'au moins une souche qui induit une augmentation de l'expression de la protéine NHE3.

La souche sélectionnée à l'issue du procédé de sélection est ainsi une souche probiotique utile dans le traitement et/ou la prévention de la diarrhée.

Par « souche probiotique », on entend désigner ici une souche d'un microorganisme vivant qui exerce un effet bénéfique sur la santé de l'hôte.

L'hôte est généralement un être humain, mais il est concevable que l'hôte puisse être un autre mammifère, comme par exemple les chiens, les chats, les ruminants, en particulier les moutons, les chèvres, les veaux, les vaches, les cervidés, les camélidés, les équidés, les suidés, en particulier les porcs et les porcelets, les léporidés, les muridés et les caviidés.

### Souches à Tester

La souche à tester par un procédé de sélection peut être n'importe quelle souche de microorganisme.

La souche à tester peut être une souche de levure. Parmi les levures pouvant être testées, on peut citer, par exemple, les levures du genre *Saccharomyces,* en particulier les espèces *Saccharomyces boulardii, Saccharomyces cerevisiae,* les levures du genre *Kluyveromyces,* en particulier les espèces *Kluyveromyces marxianus.*

La souche à tester peut être une souche de bactérie.

La souche à tester est de préférence choisie parmi les bactéries reconnues comme étant sans danger pour l'homme et/ou l'animal.

Parmi les bactéries pouvant être testées, on peut citer, par exemple, les bactéries du genre *Bifidobacterium,* en particulier les espèces *Bifidobacterium animalis, Bifidobacterium bifidum, Bifidobacterium infantis, Bifidobacterium lactis, Bifidobacterium longum,* et *Bifidobacterium breve* ; les bactéries du genre *Lactobacillus,* en particulier les espèces *Lactobacillus reuteri, Lactobacillus acidophilus, Lactobacillus bulgaricus, Lactobacillus brevis, Lactobacillus casei, Lactobacillus helveticus, Lactobacillus johnsonii, Lactobacillus plantarum, Lactobacillus paracasei, Lactobacillus rhamnosus,* et *Lactobacillus salivarius* ; les bactéries du genre *Weisella,* en particulier les espèces *Weisella cibaria, Weisella kimchii, Weisella thailandensis* ; les bactéries du genre *Bacillus,* en particulier les espèces *Bacillus subtilis, Bacillus coagulans, Bacillus amyloliquefaciens, Bacillus licheniformis, Bacillus cereus* et *Bacillus clausii* ; les bactéries du genre *Lactococcus,* en particulier les espèces *Lactococcus lactis* ; les bactéries du genre *Enterococcus,* en particulier les espèces *Enterococcus faecium, Enterococcus faecalis* ; les bactéries du genre *Streptococcus,* en particulier les espèces *Streptococcus thermophilus* ; les bactéries du genre *Escherichia,* en particulier les espèces *Escherichia coli.*

### Expression de CFTR et/ou NHE3

Un procédé de sélection décrit ici comprend la mesure de l'effet de la souche à tester sur l'expression d'au moins un transporteur impliqué dans l'absorption ou la sécrétion d'eau au niveau du côlon, en particulier sur l'expression de la protéine CFTR et/ou de la protéine NHE3.

On entend ici par « protéine CFTR », la protéine qui dans l'espèce humaine est codée par le gène *CFTR* (pour « *Cystic Fibrosis Transmembrane Conductance Regulator* ») qui est localisé sur le locus 7q31.2, dans la région q31.2 du bras long du chromosome 7. Le gène CFTR est très conservé parmi les espèces. Chez l'humain, la protéine CFTR a par exemple la séquence polypeptidique de numéro d'ordre GenBank NP_00483 qui est d'une longueur de 1480 acides aminés.

La protéine CFTR forme un canal perméable aux ions chlorure et thiocyanate des cellules épithéliales.

La protéine CFTR est exprimée au pôle apical des cellules épithéliales des canaux pancréatiques, biliaires, des cryptes intestinales, de l'arbre trachéo-bronchique, des tubules rénaux, de l'appareil génital et des glandes sudoripares.

Il a été démontré que des inhibiteurs de CFTR, comme la thiazolidinone, trouvaient application dans le traitement et/ou la prévention de diarrhées (Verkman et al., Curr. Pharm. Des., 2006, 12: 2235-2247).

Dans un procédé de sélection selon l'invention, une souche testée qui induit une diminution de l'expression de la protéine CFTR peut donc être utile dans le traitement et/ou la prévention de la diarrhée.

On entend ici par «protéine NHE3 », la protéine qui dans l'espèce humaine est codée par le gène *NHE3* (pour « *Sodium-Hydrogen Exchanger 3* », encore connu sous le nom *SLC9A3* pour « *Soluté Carrier Family 9 Member 3* »), qui est localisé sur le chromosome 13. Chez l'humain, la protéine NHE3 a par exemple la séquence polypeptidique de numéro d'ordre GenBank NP_004165 qui est d'une longueur de 834 acides aminés.

La protéine NHE3 est exprimée dans le néphron du rein et dans la membrane apicale des cellules endothéliales de l'intestin, où elle est principalement responsable du maintien de l'équilibre du sodium.

Il a été montré que des souris génétiquement modifiées pour inactiver le gène *NHE3* souffraient de diarrhée (Schultheis et al., 1998, Nat. Genet 19 : 282-285).

Dans un procédé de sélection selon l'invention, une souche testée qui induit une augmentation de l'expression de la protéine NHE3 peut donc être utile dans le traitement et/ou la prévention de la diarrhée.

La mesure de l'effet de la souche à tester sur l'expression d'un transporteur impliqué dans l'absorption ou la sécrétion d'eau au niveau du côlon, en particulier sur l'expression de la protéine CFTR et/ou de la protéine NHE3, peut être effectuée en utilisant n'importe quelle méthode connue de l'homme du métier (voir par exemple, Harlow and Lane, "Antibodies: A Laboratories Manual", 1988, Cold Spring Harbor Laboratory: Cold Spring Harbor, NY), la nature de la méthode utilisée n'étant pas un élément critique ou limitant.

En général, le taux d'expression d'une protéine peut être déterminé par une méthode directe, telle que par une méthode d'électrophorèse, par une méthode de Western-blot, par une méthode d'immunoessai, par une méthode d'immunohistochimie, par une méthode d'immunocytochimie, par une méthode de spectrométrie de masse.

Il est également possible de déterminer le taux d'expression d'une protéine par une méthode indirecte.

Une méthode indirecte peut consister à mesurer le taux de transcription du gène correspondant, par exemple par RT-PCR (transcription inverse suivie d'une amplification en chaîne par polymérase), de préférence par RT-PCR quantitative, ou par une méthode de Northern-blot.

Un autre exemple de méthode indirecte peut consister à mesurer l'activité de la protéine, l'activité de la protéine étant corrélée à la quantité de protéine fonctionnelle présente.

Le taux d'expression du transporteur, et en particulier de la protéine CFTR et/ou de la protéine NHE3, peut être déterminé par électrophorèse.

L'électrophorèse permet de séparer des protéines selon leur masse moléculaire. Il s'agit généralement d'une électrophorèse en gel de polyacrylamide contenant du laurylsulfate de sodium (appelée SDS-PAGE pour *Sodium Dodecyl Sulfate PolyAcrylamide Gel Electrophoresis*). Il peut également s'agir d'une électrophorèse bidimentionnelle, telle que l'isoélectrofocalisation qui permet de séparer les protéines selon leur masse moléculaire et leur point isoélectrique.

Le taux d'expression du transporteur, et en particulier de la protéine CFTR et/ou de la protéine NHE3, peut être déterminé par Western-blot.

Le Western-blot, appelé également technique de Western, consiste à séparer les protéines par électrophorèse sur un gel de polyacrylamide, puis à transférer les protéines depuis le gel de polyacrylamide vers une membrane support, sur laquelle les protéines d'intérêt sont repérées par utilisation d'anticorps marqués spécifiques desdites protéines.

Le taux d'expression du transporteur, et en particulier de la protéine CFTR et/ou de la protéine NHE3, peut être déterminé par un immunoessai. Par « immunoessai », on désigne ici toute technique qui utilise la réaction antigène-anticorps pour la détection et la quantification d'antigènes, d'anticorps ou de substances voisines. Un complexe anticorps-antigène peut être visualisé de plusieurs façons. Dans la plupart des cas, un anticorps est conjugué à une enzyme (ex : la peroxydase) qui peut catalyser une réaction de production de couleur (ex : coloration immunoperoxydase). Alternativement, les anticorps peuvent être marqués par un fluorophore (ex : FITC, Rhodamine, Texas Red ou Alexa Fluor). La détection du complexe anticorps-antigène se fait alors par immunofluorescence.

Des anticorps monoclonaux spécifiques de la protéine CFTR humaine et de la protéine NHE3 humaine sont connus dans l'état de la technique et sont disponibles commercialement.

Le taux d'expression du transporteur, et en particulier de la protéine CFTR et/ou de la protéine NHE3 peut être déterminé par immunohistochimie. Le terme « immunohistochimie » fait référence à une méthode basée sur l'utilisation d'anticorps pour situer une protéine dans des tissus. Le tissu est généralement fixé, lavé, puis perméabilisé. Il est ensuite mis en incubation avec un anticorps primaire qui reconnaît spécifiquement la protéine d'intérêt (par exemple ici la protéine CFTR ou la protéine NHE3). Un deuxième anticorps couplé à un système de détection est ajouté. Le système de détection est par exemple un fluorochrome ou une enzyme dont la mise en présence du substrat provoque une réaction colorée. Ce deuxième anticorps reconnaît la partie constante Fc de l'anticorps primaire et doit provenir d'une autre espèce que ce dernier. Après montage dans un milieu protégeant la fluorescence de l'échantillon s'il s'agit d'un fluorochrome ou en présence du substrat s'il s'agit d'une enzyme, le tissu est analysé au microscope à fluorescence ou confocal. Le taux d'expression de la protéine d'intérêt peut alors être déterminé par analyse densitométrique, par mesure de l'intensité de fluorescence, ou par spectrophotométrie.

Le taux d'expression du transporteur, et en particulier de la protéine CFTR et/ou de la protéine NHE3 peut être déterminé par immunocytochimie.

L'immunocytochimie est une technique similaire à l'immunohistochimie, sauf que l'échantillon de départ est une préparation cytologique au lieu d'un tissu.

Le taux d'expression du transporteur, et en particulier de la protéine CFTR et/ou de la protéine NHE3 peut être déterminé par spectrométrie de masse.

Le taux d'expression du transporteur, et en particulier de la protéine CFTR et/ou de la protéine NHE3 peut être déterminé par RT-PCR.

La RT-PCR consiste en une transcription inverse de l'ARN en ADNc, suivie d'une amplification en chaîne par polymérase (PCR). La RT-PCR est de préférence une RT-PCR quantitative qui permet de quantifier l'ARN initial correspondant à la protéine d'intérêt.

Le taux d'expression du transporteur, et en particulier de la protéine CFTR et/ou de la protéine NHE3 peut être déterminé par Northern-blot.

Le Northern-blot, appelé également transfert de Northern, est une technique consistant à séparer les molécules d'ARN en fonction de leur taille par électrophorèse sur gel, puis à transférer l'ARN depuis le gel sur une membrane où il peut être détecté par une sonde d'hybridation marquée qui est spécifique de l'ARN de la protéine à quantifier.

### Echantillon Biologique

Un procédé de sélection décrit ici comprend la mesure de l'effet de la souche à tester sur l'expression d'au moins un transporteur impliqué dans l'absorption ou la sécrétion d'eau au niveau du côlon, en particulier sur l'expression de la protéine CFTR et/ou de la protéine NHE3, dans un échantillon biologique.

Le terme « échantillon biologique », tel qu'utilisé ici, a son sens le plus large. Un échantillon biologique peut être n'importe quel tissu biologique dans lequel la protéine d'intérêt (en particulier la protéine CFTR ou la protéine NHE3) est exprimée et peut donc être détectée et quantifiée.

Les échantillons biologiques qui peuvent être utilisés dans la mise en oeuvre d'un procédé de sélection décrit ici peuvent par exemple provenir de l'intestin, les canaux pancréatiques, les canaux biliaires, les tubules rénaux, l'arbre trachéo-bronchique, l'appareil génital, les glandes sudoripares et le néphron du rein.

Préférablement, l'échantillon biologique est un échantillon qui provient de l'intestin, de préférence un échantillon du côlon.

Un échantillon tissulaire est préférablement prélevé sur un animal modèle, par exemple, par biopsie. L'animal modèle est préférablement un mammifère, par exemple, une souris, un rat, un lapin, un porc, un poulet, un singe. L'animal modèle peut être un animal transgénique.

Un échantillon biologique préféré est un échantillon tissulaire comprenant des colonocytes, c'est-à-dire des cellules de la muqueuse colique.

L'échantillon biologique peut également être un matériel biologique dérivé d'un tissu et qui exprime la protéine d'intérêt.

Le matériel biologique dérivé est par exemple choisi parmi des cellules isolées de l'échantillon tissulaire ou encore des protéines extraites de cellules isolées de l'échantillon tissulaire.

Le terme « échantillon biologique » inclut également des cellules d'une lignée cellulaire connue pour exprimer la protéine d'intérêt, en particulier la protéine CFTR et/ou la protéine NHE3.

Des exemples de lignées cellulaires exprimant la protéine CFTR incluent les cellules Caco-2 isolées du côlon, les cellules HT-29 isolées du côlon ou des cellules T84 isolées du côlon.

Des exemples de lignées cellulaires exprimant la protéine NHE3 incluent des cellules Caco-2 isolées du côlon, des cellules HT-29 isolées du côlon ou des cellules T84 isolées du côlon.

Ces lignées cellulaires sont commercialement disponibles, par exemple à l'ATCC *(American Type Culture Collection).*

L'échantillon biologique peut être un extrait protéique isolé de cellules ou de tissus exprimant la protéine d'intérêt, en particulier la protéine CFTR et/ou NHE3. Les méthodes d'extraction de protéines sont bien connues de l'homme du métier (voir par exemple, "Protein Methods", D.M. Bollag et al., 2nd Ed., 1996, Wiley-Liss; "Protein Purification Methods: A Practical Approach", E.L. Harris and S. Angal (Eds.), 1989; "Protein Purification Techniques: A Practical Approach", S. Roe, 2nd Ed., 2001, Oxford University Press; "Principles and Reactions of Protein Extraction, Purification, and Characterization", H. Ahmed, 2005, CRC Press: Boca Raton, FL). Il existe également des kits commerciaux pour l'extraction de protéines à partir de tissus ou de fluides biologiques. De tels kits sont, par exemple, vendus par BioRad Laboratories (Hercules, CA), BD Biosciences Clontech (Mountain View, CA), Chemicon International, Inc. (Temecula, CA), Calbiochem (San Diego, CA), Pierce Biotechnology (Rockford, IL), et Invitrogen Corp. (Carlsbad, CA). L'homme du métier sait sélectionner le kit le plus adapté à la situation.

### Effet de la Souche à tester

Dans un procédé de sélection décrit ici, l'effet de la souche à tester sur l'expression d'un transporteur impliqué dans l'absorption ou la sécrétion d'eau au niveau du côlon, en particulier de la protéine CFTR et/ou de la protéine NHE3, est déterminé en comparant le taux d'expression de la protéine d'intérêt mesuré dans un échantillon biologique obtenu d'un animal modèle qui a été traité par la souche à tester et le taux d'expression de la protéine d'intérêt mesuré dans un échantillon biologique (de même nature que le précédent) obtenu d'un animal modèle (de même nature que le précédent) qui n'a pas été traité par la souche à tester. Ce dernier animal est appelé animal contrôle.

Ainsi, par exemple, dans la mise en oeuvre d'un procédé de sélection décrit ici dans lequel l'échantillon biologique provient d'un animal modèle, une quantité donnée de cellules obtenues par culture de la souche à tester est administrée à l'animal modèle.

La quantité de cellules peut être administrée en une seule fois.

Alternativement, la quantité de cellules peut être administrée en plusieurs fois, soit dans la même journée ou sur plusieurs jours ou semaines.

L'administration des cellules obtenues par culture de la souche à tester peut être effectuée par n'importe quelle méthode d'administration adaptée à l'animal modèle utilisé. Par exemple, l'administration peut se faire par injection, en particulier par injection intra-gastrique, d'une solution ou d'une suspension contenant un véhicule approprié et une quantité donnée de cellules obtenues par culture de la souche à tester. On administre à l'animal contrôle le même volume d'une solution ne contenant que le véhicule.

De même, dans la mise en oeuvre d'un procédé de sélection décrit ici dans lequel l'échantillon biologique est une lignée cellulaire, l'effet de la souche à tester est déterminé en comparant le taux d'expression de la protéine d'intérêt mesuré sur les cellules de la lignée cellulaire qui ont été traitées par la souche à tester et le taux d'expression de la protéine d'intérêt mesuré dans des cellules (de la même lignée cellulaire) qui n'ont pas été traitées par la souche à tester. Ces dernières cellules sont appelées cellules contrôles.

Ainsi, par exemple, dans la mise en oeuvre d'un procédé de sélection décrit ici dans lequel l'échantillon biologique est une lignée cellulaire, des cellules d'une lignée cellulaire sont mises en incubation avec une quantité donnée d'une solution ou d'une suspension contenant un véhicule approprié et une quantité donnée de cellules obtenues par culture de la souche à tester. Les cellules contrôles de la lignée cellulaire sont mises en incubation dans les mêmes conditions en présence du même volume d'une solution ne contenant que le véhicule.

Les procédés de culture d'une souche, qu'il s'agisse d'une souche de levure ou d'une souche de bactérie, sont connues dans l'art, et l'homme du métier sait optimiser les conditions de culture pour chaque souche en fonction de sa nature.

De même, l'homme du métier sait déterminer les quantités optimales de cellules obtenues par culture de la souche à tester pour l'administration à un animal modèle ou pour la mise en incubation avec des cellules d'une lignée cellulaire.

Par exemple, des cellules de *Bacillus subtilis* sont obtenues par culture d'une souche de *Bacillus subtilis* dans un milieu de culture, en particulier comme décrit dans le livre Biotechnologie, 5e édition, R. Scriban, Edition Tec. & Doc., 1999, ISBN : 2-7430-0309-X.

Typiquement, un procédé de production de cellules de *Bacillus subtilis* par culture d'une souche de *Bacillus subtilis* comprend les étapes de :
- ensemencement d'un milieu de culture avec un inoculum de la souche de *Bacillus subtilis,*
- culture en condition aérobie, pour obtenir la multiplication des cellules,
- séparation de la biomasse de son milieu de culture, pour obtenir des cellules de *Bacillus subtilis.*

Les cellules de *Bacillus subtilis* ainsi obtenues sont majoritairement sous forme végétative.

L'expression « majoritairement sous forme végétative » signifie qu'au moins 70% des cellules sont sous forme végétative, de préférence au moins 80%, et plus préférentiellement au moins 90%.

La « forme végétative » d'une bactérie désigne la forme d'une bactérie placée dans des conditions favorables.

Un exemple de conditions favorables est un milieu de culture non limitant à une température et un pH favorables à la multiplication bactérienne.

Un milieu de culture non limitant contient tous les éléments nutritifs nécessaires à la multiplication des cellules.

Le procédé de production de cellules de *Bacillus subtilis* peut également comprendre une étape intermédiaire de mise en condition défavorable des cellules, entre l'étape de culture en condition aérobie et l'étape de séparation de la biomasse. Les cellules de *Bacillus subtilis* obtenues à l'issue du procédé de production sont ainsi majoritairement sous forme sporulée.

L'expression « majoritairement sous forme sporulée » signifie qu'au moins 70% des cellules sont sous forme sporulée, de préférence au moins 80%, et plus préférentiellement au moins 90%.

La « forme sporulée » d'une bactérie désigne la forme d'une bactérie placée dans des conditions défavorables. La forme sporulée est ainsi une forme de résistance qui lui permet de résister à un milieu difficile, tel que par exemple un manque de nutriments, c'est-à-dire un milieu nutritif limitant, un stress hydrique, une variation importante du pH ou de la température, ou encore à la traversée du tube digestif.

La mise en condition défavorable des cellules de bactéries est par exemple obtenue par : un non renouvèlement du milieu de culture des bactéries, un arrêt de l'alimentation en milieu de culture, l'utilisation d'un milieu de culture limitant, un changement de température, un changement de pH ou leur combinaison.

Le procédé de production de cellules de *Bacillus subtilis* peut également comprendre une étape ultérieure de séchage des cellules, pour obtenir des cellules de *Bacillus subtilis* sous forme sèche.

L'expression « cellules de *Bacillus subtilis* sous forme sèche » signifie que la biomasse obtenue à l'issue du procédé de production des cellules de *Bacillus subtilis* comprend plus de 90% de matières sèches, de préférence plus de 95 % de matières sèches.

Le séchage est par exemple un séchage par lyophilisation, un séchage sur lit fluidisé ou un séchage par atomisation.

### Identification d'une Souche Probiotique pour le Traitement et/ou la Prévention de la Diarrhée

La comparaison du taux d'expression de la protéine d'intérêt mesuré dans l'échantillon traité par la souche à tester et du taux d'expression de la protéine d'intérêt mesuré dans l'échantillon contrôle permet de déterminer si la souche testée induit une diminution ou une augmentation de l'expression de la protéine d'intérêt ou si elle n'a aucun effet sur cette expression.

Comme indiqué plus haut, une souche dont on détermine par un procédé de sélection décrit ici qu'elle induit une diminution de l'expression d'au moins un transporteur impliqué dans la sécrétion d'eau au niveau du côlon et/ou une augmentation de l'expression d'au moins un transporteur impliqué dans l'absorption d'eau au niveau du côlon est une souche probiotique qui peut être utile dans le traitement et/ou la prévention de la diarrhée.

Les Inventeurs ont démontré qu'une telle souche probiotique est capable d'induire une absorption de l'eau présente en excès au niveau du côlon, et trouve donc application dans le traitement et/ou la prévention de la diarrhée.

Par « diminution de l'expression d'un transporteur impliqué dans la sécrétion d'eau au niveau du côlon », on entend désigner une diminution significative de l'expression dudit transporteur par rapport à son expression en l'absence de la souche à tester.

Le caractère « significatif » de cette diminution est déterminé par analyse statistique.

Lorsque l'effet sur l'expression du transporteur est mesuré par immunohistochimie, une « diminution de l'expression du transporteur » correspond à une diminution significative de l'intensité de fluorescence / µm² correspondant à l'expression dudit transporteur par rapport à celle en l'absence de la souche à tester.

Par « augmentation de l'expression d'un transporteur impliqué dans l'absorption d'eau au niveau du côlon », on entend désigner une augmentation significative de l'expression dudit transporteur par rapport à son expression en l'absence de la souche à tester.

Le caractère « significatif » de cette augmentation est déterminé par analyse statistique.

Lorsque l'effet sur l'expression du transporteur est mesuré par immunohistochimie, une « augmentation de l'expression du transporteur » correspond à une augmentation significative de l'intensité de fluorescence / µm2 correspondant à l'expression dudit transporteur par rapport à celle en l'absence de la souche à tester.

Comme le reconnaîtra l'homme du métier, l'identification d'une souche probiotique utile pour traiter et/ou prévenir les diarrhées peut être réalisée en utilisant uniquement un procédé de sélection décrit ici. Le procédé de sélection peut être effectué en utilisant un seul type d'échantillon biologique, par exemple un animal modèle donné, ou en utilisant plusieurs types d'échantillons biologiques, par exemple une lignée cellulaire, puis au moins un animal modèle.

Alternativement, l'identification d'une souche probiotique capable d'agir sur l'absorption d'eau au niveau du côlon peut être réalisée en utilisant un procédé de sélection décrit ici puis en complétant cette sélection par une série de tests et essais cliniques, bien connus de l'homme du métier.

### II - Utilisation d'une Souche Probiotique dans le Traitement et/ou la Prévention de la Diarrhée

Toute souche probiotique identifiée par un procédé de sélection comme étant capable d'induire une absorption de l'eau présente en excès au niveau du côlon peut être utile dans le traitement et/ou la prévention de la diarrhée. Une telle souche probiotique peut être utilisée dans la fabrication d'un médicament pour le traitement et/ou la prévention de la diarrhée.

Comme indiqué plus haut, la souche probiotique peut être n'importe quelle souche de microorganisme, en particulier une souche de levure ou une souche de bactérie, comme celles précédemment citées.

En particulier, les Inventeurs ont démontré que la souche de *Bacillus subtilis* CU1 déposée à la CNCM le 25 octobre 2001 sous le numéro I-2745 est une souche probiotique qui agit sur l'absorption d'excès d'eau au niveau du côlon, et peut donc être utile pour traiter et/ou prévenir les diarrhées.

Cette souche a été décrite dans le document FR 2 837 835 qui divulgue un adjuvant vivant comprenant ladite souche pour une immunisation au niveau de la muqueuse gastro-intestinale.

Les cellules de levure ou cellules de bactérie obtenues par culture d'une souche probiotique identifiée par un procédé de sélection décrit ici sont utiles dans le traitement et/ou la prévention de la diarrhée.

Comme indiqué ci-dessus, les conditions de culture d'une souche sont généralement connues dans l'art ou sont facilement déterminables par l'homme du métier.

En particulier, les cellules obtenues par culture de la souche de *Bacillus subtilis* CU1 déposée à la CNCM le 25 octobre 2001 sous le numéro I-2745, sont utiles dans le traitement et/ou la prévention de la diarrhée.

Les « cellules obtenues par culture de la souche de *Bacillus subtilis* CU1 déposée à la CNCM le 25 octobre 2001 sous le numéro I-2745 » sont appelées « cellules CU1 » par la suite.

La production de cellules CU1 peut être réalisée tel que décrit précédemment.

Les cellules CU1 utilisées dans le traitement et/ou la prévention de la diarrhée sont des cellules sous forme sporulée et/ou sous forme végétative.

Préférablement, les cellules CU1 sont majoritairement sous forme sporulée.

Préférablement, les cellules CU1 utilisées dans le traitement et/ou la prévention de la diarrhée sont sous forme sèche.

Plus préférablement encore, les cellules CU1 utilisées dans le traitement et/ou la prévention de la diarrhée sont majoritairement sous forme sporulée et sous forme sèche.

Tout extrait de cellule de levure probiotique ou de cellule de bactérie probiotique et tout filtrat de culture d'une souche probiotique, possédant la capacité d'induire une diminution de l'expression de la protéine CFTR et/ou une augmentation de l'expression de la protéine NHE3, est capable d'induire une absorption d'eau en excès au niveau du côlon.

Une méthode de traitement et/ou de prévention de la diarrhée comprend l'administration, au patient, d'une quantité efficace de cellules de levure ou de cellules de bactérie obtenues par culture d'une souche probiotique identifiée par un procédé de sélection décrit ici. La quantité efficace de cellules de levure ou de cellules de bactérie, qui peut être administrée en une ou plusieurs doses, peut être déterminée par le médecin. La quantité exacte à administrer peut varier d'un patient à un autre, en fonction de l'âge, du poids, de la condition générale du patient, de la nature et de la gravité de la diarrhée, etc. La quantité exacte à administrer peut également varier en fonction de l'effet thérapeutique désiré (c'est-à-dire prévenir une diarrhée ou traiter une diarrhée).

La voie d'administration, orale ou parentérale, peut être choisie en fonction de la nature de la diarrhée et/ou en fonction de l'âge et de la condition générale du patient.

En cas de prévention d'une diarrhée, les cellules de levure ou les cellules de bactérie obtenues par culture d'une souche probiotique identifiée par le procédé de sélection décrit ici sont administrées avant l'apparition des symptômes.

En cas de traitement d'une diarrhée, les cellules de levure ou les cellules de bactérie obtenues par culture d'une souche probiotique identifiée par le procédé de sélection décrit ici sont administrées après l'apparition des symptômes.

D'une manière générale, toute diarrhée peut être traitée en utilisant une souche probiotique identifiée par le procédé de sélection décrit ici ou des cellules obtenues par culture de ladite souche probiotique, y compris les diarrhées aiguës et les diarrhées chroniques.

On entend par « diarrhée aigüe », une diarrhée dont les symptômes durent moins de deux semaines, et par « diarrhée chronique », une diarrhée dont les symptômes durent au moins deux semaines, de préférence au moins un mois.

Les diarrhées aiguës incluent, par exemple, les gastro-entérites virales, les gastro-entérites bactériennes, et les toxi-infections alimentaires (par exemple les salmonelloses, shigelloses, les infections à *Staphylococcus aureus,* les infections à *Bacillus cereus,* les infections à *Escherichia Coli*).

Les diarrhées chroniques incluent, par exemple, le syndrome de malabsorption, les gastroentéropathies (maladie coeliaque, maladie de Whipple, maladie de Crohn, etc...), la diarrhée motrice (c'est-à-dire une diarrhée due à une accélération du transit intestinal), la diarrhée sécrétoire (c'est-à-dire une diarrhée due à une accélération de la sécrétion hydro-électrolytique au niveau de l'intestin grêle et/ou du côlon), la diarrhée osmotique (c'est-à-dire une diarrhée qui découle de la présence, dans le tractus gastro-intestinal, de solutés qui sont mal absorbés et produisent un effet osmotique).

D'autres diarrhées qui peuvent être traitées en utilisant une souche probiotique identifiée par un procédé de sélection décrit ici ou des cellules obtenues par culture d'une telle souche probiotique, incluent les diarrhées dues à l'anxiété ou aux émotions intenses, les diarrhées dues aux intolérances alimentaires (par exemple, lactose dans le lait de vache, sorbitol, gluten, etc.), les diarrhées dues à certains traitements comme la magnésothérapie, la radiothérapie ou la chimiothérapie ou à certaines chirurgies comme la gastrectomie ou la résection iléale, les diarrhées dues à une surcharge ou un irritation de l'estomac, des intestins, du côlon, etc., les diarrhées dues au sevrage de stupéfiants tels que l'héroïne, les diarrhées dues à certains médicaments tels que les antibiotiques.

D'autres exemples de diarrhées qui peuvent être traitées en utilisant une souche probiotique identifiée par un procédé de sélection décrit ici, ou des cellules obtenues par culture d'une telle souche probiotique, incluent les diarrhées qui accompagnent les crises de foie ou indigestions, les diarrhées qui résultent de la surconsommation de certains aliments laxatifs, et les diarrhées du sportif.

Dans certains cas, la diarrhée traitée en utilisant une souche probiotique identifiée par un procédé de sélection décrit ici, ou des cellules obtenues par culture d'une telle souche probiotique n'est pas une diarrhée induite par administration d'un antibiotique, ou plus généralement par l'administration d'un médicament.

Dans d'autres cas, la diarrhée traitée en utilisant une souche probiotique identifiée par un procédé de sélection décrit ici, ou des cellules obtenues par culture d'une telle souche probiotique n'est pas une diarrhée d'origine infectieuse.

### III - Compositions

Est également écrite ici une composition pour son utilisation dans le traitement et/ou la prévention de la diarrhée, la composition comprenant au moins une souche probiotique identifiée par un procédé de sélection telle que défini plus haut, ou plus spécifiquement des cellules obtenues par culture d'une telle souche. En particulier, une composition préférée comprend une quantité efficace de cellules obtenues par culture de la souche de *Bacillus subtilis* CU1 déposée à la CNCM le 25 octobre 2001 sous le numéro I-2745.

La composition peut être une composition alimentaire, un complément alimentaire ou une composition pharmaceutique.

Une composition alimentaire désigne n'importe quel type d'aliment, boisson ou confiserie.

La composition alimentaire peut par exemple être une boisson, une barre de céréales, un chewing-gum, du chocolat, un produit laitier, tel qu'un produit laitier fermenté, un produit fermenté d'origine végétale.

On entend par « complément alimentaire » une denrée alimentaire dont le but est de compléter le régime alimentaire normal. Un complément alimentaire constitue une source concentrée de nutriments ou d'autres substances ayant un effet nutritionnel ou physiologique, seuls ou combinés.

Un complément alimentaire est commercialisé sous forme de dose, à savoir les formes de présentation telles que gélule, pastille, comprimé, pilule et autres formes similaires, sachet de poudre, ampoule de liquide, flacon muni d'un compte-goutte et autres formes analogues de préparations liquides ou en poudres destinées à être prises en unités mesurées de faible quantité.

Une composition est par exemple appropriée pour une utilisation quotidienne des cellules obtenues par culture de la souche probiotique dans une quantité de 1.10⁸ UFC à 1.10¹¹ UFC, de préférence dans une quantité de 1.10⁹ UFC à 1.10¹⁰ UFC lorsqu'elle est destinée à un usage humain.

Une composition est par exemple appropriée pour une utilisation quotidienne des cellules obtenues par culture de la souche probiotique dans une quantité de 1.10⁵ UFC à 1.10¹¹ UFC, de préférence dans une quantité de 1.10⁶ UFC à 1.10¹⁰ UFC lorsqu'elle est destinée à un usage vétérinaire.

Le terme UFC désigne une Unité Formant Colonie.

### IV - Compositions Pharmaceutiques

En raison de son action directe au niveau des transporteurs impliqués dans la régulation du fluide intestinal, une souche probiotique identifiée par un procédé de sélection décrit ici, ou plus spécifiquement des cellules de levure ou des cellules de bactérie obtenues par culture d'une telle souche, peuvent être utilisées comme agent thérapeutique.

De telles cellules de levure ou de bactérie peuvent être administrées comme telles ou sous la forme d'une préparation ou composition pharmaceutique en présence d'au moins un véhicule ou excipient physiologiquement acceptable. On entend ici par « véhicule ou excipient physiologiquement acceptable » tout milieu ou additif qui n'interfère pas avec l'efficacité de l'activité biologique du principe actif (ici, les cellules de levure ou de bactérie), et qui n'est pas excessivement toxique pour le patient ou sujet, aux concentrations auxquelles il est administré. Un véhicule ou excipient physiologiquement acceptable peut être un véhicule ou excipient approprié à l'administration aux humains et/ou aux animaux (en particulier aux mammifères).

Les compositions pharmaceutiques peuvent être administrées en utilisant toute combinaison de dosage et de voie d'administration efficace pour obtenir l'effet thérapeutique désiré. La quantité exacte à administrer peut varier d'un patient à un autre, en fonction de l'âge, du poids, de la condition générale du patient, de la nature et de la gravité de la diarrhée, etc. La voie d'administration (orale ou parentérale) peut être choisie en fonction de la nature de la diarrhée et/ou en fonction de l'âge et/ou de la santé du patient.

A titre d'exemple, une composition pharmaceutique telle que définie ci-dessus peut être destinée pour une utilisation quotidienne de cellules obtenues par culture de ladite souche probiotique dans une quantité de 1.10⁸ UFC à 1.10¹¹ UFC, de préférence dans une quantité de 1.10⁹ UFC à 10¹⁰ UFC, lorsqu'elle est destinée à un usage humain.

Alternativement, une composition pharmaceutique telle que définie ci-dessus peut être destinée pour une utilisation quotidienne de cellules obtenues par culture de ladite souche probiotique dans une quantité de 1.10⁵ UFC à 1.10¹¹ UFC, de préférence dans une quantité de 1.10⁶ UFC à 1.10¹⁰ UFC, lorsqu'elle est destinée à un usage vétérinaire.

La formulation d'une composition pharmaceutique peut varier en fonction de la voie d'administration et du dosage pour lesquels la composition est destinée à être utilisée. Après formulation avec au moins un véhicule ou excipient physiologiquement acceptable, une composition pharmaceutique peut être sous toute forme appropriée pour l'administration à un mammifère, en particulier l'homme, par exemple sous la forme de tablettes, comprimés, dragées, capsules, sirops, onguents, solutions injectables, suppositoires, etc. L'homme de l'art sait sélectionner les véhicules et excipients les plus appropriés à la préparation d'un type donné de formulation. Ainsi, par exemple, les excipients tels que l'eau, le 2,3-butanediol, la solution de Ringer, les solutions isotoniques de chlorure de sodium, les mono ou diglycérides synthétiques, et l'acide oléique sont souvent utilisés pour la formulation de préparations injectables. Les compositions liquides, y compris les émulsions, microémulsions, solutions, suspensions, sirops, élixirs, etc., peuvent être formulés en présence de solvants, d'agents solubilisants, d'émulsifiants, d'huiles, d'acides gras, et d'autres additifs comme des agents de suspension, des conservateurs, des édulcorants, des arômes, des agents viscosifiants, des colorants, etc. Les compositions solides pour l'administration par voie orale peuvent être formulées en présence d'un excipient inerte comme le citrate de sodium, et éventuellement d'additifs tels que des agents liants, des agents humectants, des agents de désintégration, des accélérateurs d'absorption, des agents lubrifiants, etc.

Une composition pharmaceutique peut être formulée pour une libération immédiate du ou des principes actifs, en particulier des cellules de levure ou des cellules de bactérie obtenues par culture de la souche probiotique. Alternativement, une composition pharmaceutique peut être formulée pour une libération prolongée ou ciblée du ou des principes actifs ou pour une protection du ou des principes actifs, par exemple vis-à-vis de l'acidité et des enzymes gastriques.

Il est ainsi possible d'utiliser des enrobages résistants au pH et/ou à l'action des enzymes gastriques, des enrobages sensibles au pH et/ou à des actions enzymatiques, ou des enrobages bioadhésifs qui s'accrochent aux parois de l'estomac ou de l'intestin, ou encore en utilisant des systèmes d'encapsulation.

Les compositions pharmaceutiques peuvent, en outre, contenir au moins un principe actif pharmaceutique supplémentaire (c'est-à-dire, en plus des cellules de levure ou de bactérie obtenues par culture de la souche probiotique).

On entend par « principe actif pharmaceutique », tout composé ou substance dont l'administration a un effet thérapeutique ou un effet bénéfique à la santé ou condition générale d'un patient ou d'un sujet auquel il est administré.

Ainsi, un principe actif pharmaceutique peut être actif contre la diarrhée que l'on veut prévenir ou soigner par administration de la composition pharmaceutique ; ou peut être actif contre une condition ou un symptôme associé à la diarrhée (par exemple, une fièvre, un vomissement ou des crampes au ventre) ; ou encore peut accroître la disponibilité et/ou l'activité du ou des principes actifs de la composition pharmaceutique.

Des exemples de principes actifs pharmaceutiques qui peuvent être présents dans une composition incluent, sans limitation, des anti-inflammatoires, des antibiotiques, des agents antipyrétiques, des agents anti-émétiques, des agents antihistaminiques, des vitamines, des agents anti-spasmodiques, etc.

Préférablement, l'autre principe actif pharmaceutique n'est pas une souche de *Bacillus subtilis* ou des cellules obtenues par culture d'une telle souche, de préférence n'est pas une souche de bactérie ou des cellules obtenues par culture d'une telle souche, plus préférentiellement n'est pas une souche probiotique ou des cellules obtenues par culture d'une telle souche.

Un exemple de composition pharmaceutique est une composition comprenant des cellules de levure ou de bactérie obtenues par culture de la souche probiotique, comme seul principe actif pharmaceutique.

Préférablement, la composition contient des cellules obtenues par culture de la souche de *Bacillus subtilis* CU1 déposée à la CNCM le 25 octobre 2001 sous le numéro I-2745, à l'exclusion de toute autre souche de bactérie.

Plus préférablement encore, la composition selon l'invention contient des cellules obtenues par culture de la souche de *Bacillus subtilis* CU1 déposée à la CNCM le 25 octobre 2001 sous le numéro I-2745, à l'exclusion de toute autre souche de bactérie ou de levure.

De manière encore plus préférée, la composition selon l'invention contient des cellules obtenues par culture de la souche de *Bacillus subtilis* CU1 déposée à la CNCM le 25 octobre 2001 sous le numéro I-2745, comme seul principe actif pharmaceutique.

A moins qu'ils ne soient définis d'une autre manière, tous les termes techniques et scientifiques utilisés dans la description ont la même signification que celle couramment comprise par un spécialiste ordinaire du domaine auquel appartient cette invention.

### Exemples

Les exemples suivants décrivent certains modes de réalisation de la présente invention. Cependant, il est entendu que les exemples ne sont présentés qu'à titre illustratif et ne limitent en aucun cas la portée de l'invention.

### Légende des Figures

**Figure 1****.** Effet du traitement avec la souche *Bacillus subtilis* CU1 sur l'expression colique de la protéine CFTR. L'intensité de fluorescence / µm² est donnée pour le contrôle (en blanc) et après traitement avec CU1 (en noir). * p<0,005
**Figure 2****.** Effet du traitement avec la souche *Bacillus subtilis* CU1 sur l'expression colique de la protéine NHE3. L'intensité de fluorescence / µm² est donnée pour le contrôle (en blanc) et après traitement avec CU1 (en noir). * p<0,005
**Figure 3****.** Absence d'effet du traitement avec la souche *Lactobacillus plantarum* sur l'expression colique de la protéine CFTR. L'intensité de fluorescence / µm² est donnée pour le contrôle (en blanc) et après traitement avec LR (en noir). * p<0,005
**Figure 4****.** Absence d'effet du traitement avec la souche *Lactobacillus plantarum* sur l'expression colique de la protéine NHE3. L'intensité de fluorescence / µm² est donnée pour le contrôle (en blanc) et après traitement avec LR (en noir). * p<0,005
**Figure 5****.** Eau excrétée (en mg) dans les fèces dans les 60 minutes (en noir) et entre 60 et 120 minutes (en blanc) après administration intraveineuse de LPS (15 mg/kg) ou d'eau saline aux souris (moyenne ± SEM, n=10-20). T- : souris non traitées par CU1 ayant reçu une injection d'eau saline ; T+ : souris non traitées avec CU1 ayant reçu une injection de LPS ; T-/CU1 : souris traitées avec CU1 ayant reçu une injection d'eau saline, T+/CU1 : souris traitées avec CU1 ayant reçu une injection de LPS. * P<0,05 vs T-; † P<0,05 vs T+

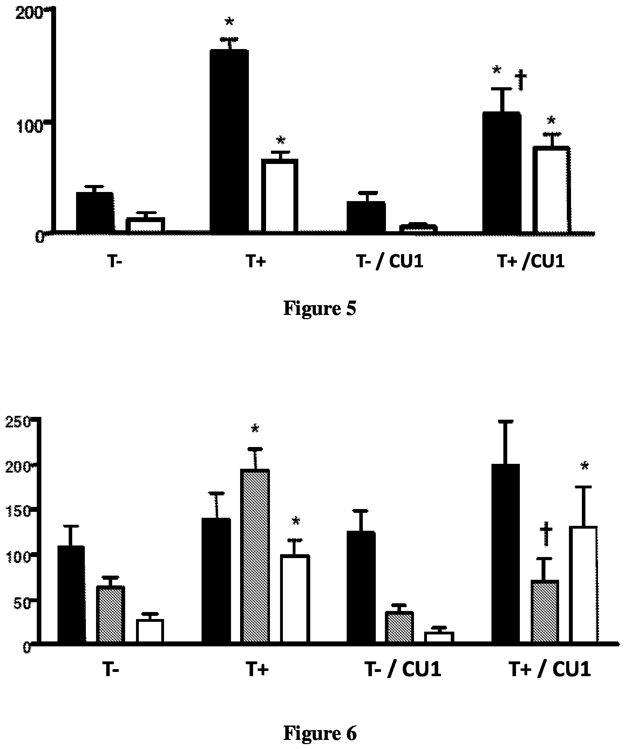

**Figure 6****.** Eau excrétée (en mg) dans les fèces dans les 60 minutes (en noir), entre 60 et 120 minutes (en gris) et entre 120 et 180 minutes (en blanc) après administration intra-gastrique d'huile de ricin (200 µL) ou d'eau saline aux souris (moyenne ± SEM, n=10-19). T- : souris non traitées par CU1 ayant reçu une injection d'eau saline ; T+ : souris non traitées avec CU1 ayant reçu une injection d'huile de ricin ; T-/CU1 : souris traitées avec CU1 ayant reçu une injection d'eau saline, T+/CU1 : souris traitées avec CU1 ayant reçu une injection d'huile de ricin. * P<0,05 vs T- ; † P<0,05 vs T+

### Exemple 1 : Sélection de Souches Probiotiques Destinées à la Prévention et/ou au Traitement de la Diarrhée

### Matériel et Méthodes

### Souches Probiotiques Testées.

Les souches testées sont les suivantes : une souche de *Lactobacillus plantarum* et la souche *Bacillus subtilis* CU1.

Les cellules de *Bacillus subtilis* CU1 (ci-après CU1) sont utilisées sous forme sporulée et sèche.

Les cellules de *Lactobacillus plantarum* (ci-après LR) sont utilisées sous forme végétative et lyophilisée.

### Mesure de l'Effet d'une Souche Probiotique sur l'Expression de CFTR et de NHE3.

Quatre souris ont été traitées 2 semaines avec chacune 10⁹ UFC / jour de CU1.

Quatre souris ont été traitées 2 semaines avec chacune 10⁹ UFC / jour de LR.

Quatre souris non traitées avec CU1 sont utilisées comme contrôle.

L'expression de CFTR et NHE3 a été mesurée par immunohistochimie sur des colonocytes issus de souris traitées avec CU1 et de souris contrôle, comme décrit ci-après.

Immédiatement après le sacrifice des souris, des échantillons du côlon proximal (1 cm de longueur) sont lavés avec une solution de NaCl 0,9%, fixés dans une solution tampon à 4% de formaldéhyde (6 h) et immergés pendant 24 h dans une solution de saccharose à 30% à 4°C.

Les échantillons sont fixés dans un milieu Tissue Tek (Euromedex, Souffelweyersheim, France) et congelés dans de l'isopentane à -45°C. Des coupes cryostat sont post-fixées avec de l'acétone (10 minutes, -20°C) et hydratées dans une solution de tampon phosphate salin (PBS) - lait. Après mise en incubation dans une solution bloquante (PBS avec 0,25% de Triton X-100 contenant 0,1% d'albumine de sérum bovine (BSA)), les coupes sont mises en incubation sur la nuit à 4°C avec un anticorps polyclonal de lapin anti-CFTR de souris (1/100), anti-NHE3 de souris (1/200) ou anti-TLR4 de souris (1/100) (Abcam, Cambridge, UK). Les coupes sont ensuite mises en incubation pendant 1 h à température ambiante avec un anticorps d'âne anti-lapin marqué avec FITC (1/2000, Uptima, Montluçon, France). Les coupes sont ensuite montées dans le milieu « Vectashield HardSet mounting médium » (Abcys, Les Ulis, France) et sont examinées sous un microscope à fluorescence Nikon 90i (Nikon, Champigny-sur-Marne, France). L'intensité de fluorescence / µm² de l'immunomarquage est mesurée avec le logiciel Nis-Elements Ar (Nikon). Les analyses sont effectuées sur cinq coupes d'un groupe de 4 souris.

### Résultats

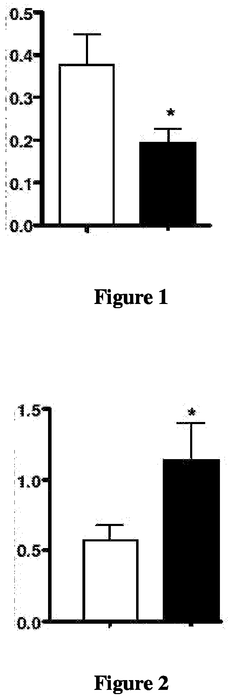

Parmi les souches probiotiques testées, seule la souche de *Bacillus subtilis* CU1 agit sur l'expression des deux transporteurs CFTR et NHE3 qui sont impliqués dans les mouvements d'eau à travers la muqueuse colique. En effet, comme le montre la Figure 1, le traitement des souris pendant deux semaines avec la souche de *Bacillus subtilis* CU1 a conduit à une réduction significative de l'expression de CFTR sur le pôle apical des colonocytes dans le côlon de souris saines. Comme le montre la Figure 2, le traitement des souris pendant 2 semaines avec la souche de *Bacillus subtilis* CU1 a conduit à une augmentation significative de l'expression de NHE3 sur le pôle apical des colonocytes dans le côlon de souris saines.

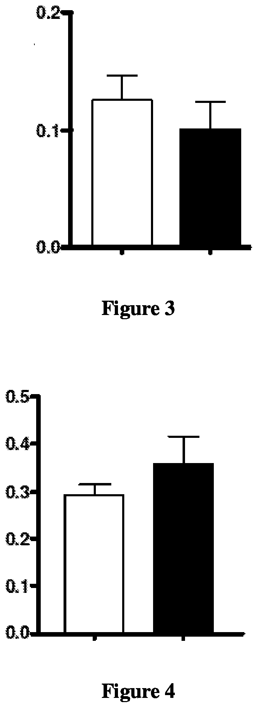

Par contre, comme le montrent les Figures 3 et 4, le traitement avec la souche de *L. plantarum* n'a modifié significativement l'expression ni de CFTR ni de NHE3.

### Exemple 2 : Utilisation de la Souche Probiotique CU1 dans la Prévention et/ou le Traitement de la Diarrhée

### Matériel et Méthodes

### Modèle de Diarrhée Induite au LPS.

Une dose de 10⁹ UFC/jour/animal de la souche probiotique à évaluer a été administrée par voie intra-gastrique chez des souris mâles DBA/2 pendant 14 jours.

Les souris du groupe contrôle (12 souris) ont ensuite été traitées avec 200 µL d'eau administrée par voie intraveineuse et les souris du groupe test (12 souris) ont reçu une administration de lipopolysaccharide (LPS) de *Salmonella enteritidis* (Sigma, Saint Quentin Fallavier, France) en intraveineuse à une dose de 15 mg/kg, afin d'induire une diarrhée. Les souris sont placées dans des cages individuelles avec le bas couvert d'une feuille d'aluminium permettant la collection des fèces toutes les 30 minutes pendant 120 minutes après l'injection de LPS. Les matières fécales collectées sont pesées et chauffées à 100°C pendant 24 h et pesées à nouveau. La différence entre la masse humide de matières fécales (avant chauffage) et la masse sèche correspond à l'excrétion d'eau. La diarrhée est évaluée par rapport à l'excrétion d'eau totale après administration du LPS.

### Modèle de Diarrhée Induite par L'huile de Ricin.

Une dose de 10⁹ UFC/jour/animal de la souche probiotique à évaluer a été administrée par voie intra-gastrique chez des souris mâles NMRI pendant 14 jours.

Les souris du groupe contrôle (12 souris) ont été traitées avec 200 µL d'eau administrée par voie intra-gastrique. 200 µL d'huile de ricin (Sigma) ont été administrés par voie intra-gastrique aux souris mâles NMRI du groupe «test» (12 souris), afin d'induire une diarrhée. Les souris sont placées dans des cages individuelles avec le bas couvert d'une feuille d'aluminium permettant la collection des fèces toutes les 30 minutes pendant 180 minutes après l'administration d'huile de ricin. Les matières fécales collectées sont pesées et chauffées à 100°C pendant 24 h et pesées à nouveau. La différence entre la masse humide de matières fécales (avant chauffage) et la masse sèche correspond à l'excrétion d'eau. La diarrhée est évaluée par rapport à l'excrétion d'eau totale après administration de l'huile de ricin.

### Modèle de Diarrhée Induite par un Antibiotique.

Un modèle animal de diarrhée induite par antibiotiques a été mis en place avec utilisation d'un nombre réduit d'antibiotiques (à partir de la publication Chen et al., 2008, Gastroenterology, 135(6): 1984-92). L'administration antibiotique induit une dysbiose (déséquilibre) de la flore ainsi qu'une réponse immunitaire et une altération du nombre de cellules productrices de mucus par l'intestin de la souris. Le traitement antibiotique peut induire notamment l'apparition de *Clostridium difficile.*

Le traitement préventif de la souche CU1 a été testé dans ce modèlebà raison de 10⁹ UFC/jour/animal pendant 14 jours et 18 jours sur des groupes de 5 souris. Après 7 jours d'administration de la souche CU1, un mélange de 5 antibiotiques est donné dans l'eau de boisson pendant 3 jours, puis le lendemain les souris traitées aux antibiotiques reçoivent une injection intra-péritonéale de clindamycine. L'effet des traitements est observé aux jours 14 et 18 du début de l'expérience. Les souris traitées avec la souche CU1 reçoivent la souche tout au long de l'essai.

### Résultats

La souche de *Bacillus subtilis* CU1 a été testée dans différents modèles de diarrhée chez la souris.

### Propriétés Anti-diarrhée de la Souche de Bacillus subtilis CU1.

Comme le montrent les Figures 4 et 5, la souche CU1 prévient significativement la diarrhée induite par le LPS (Figure 5) et par l'huile de ricin (Figure 6).

En effet, l'administration de cellules CU1 diminue significativement l'eau excrétée dans les fèces pendant la première heure après administration de LPS (sur 4 essais, la diminution était de 35%, 38%, 36% et 40%).

L'administration de cellules CU1 diminue significativement l'eau excrétée dans les fèces pendant la 2^{ème} heure dans le modèle de diarrhée induite par l'huile de ricin (sur 3 essais, la diminution était de 63%, 46%, 44%).

La souche CU1 a également un effet préventif sur la diarrhée induite par l'administration d'antibiotiques. Cet effet est observé après traitement préventif avec des cellules CU1 : les souris présentent alors une normalisation du microbiote intestinal et une amélioration des altérations occasionnées par les antibiotiques au niveau des cellules de la muqueuse intestinale productrice de mucus et sur les macrophages.

### Comparaison avec l'Effet du Lopéramide.

Un traitement préventif par la souche CU1 pendant 15 jours réduit la diarrhée induite par le LPS ou l'huile de ricin avec la même efficacité que le lopéramide, molécule anti-diarrhéique de référence, administré en dose unique (1 mg/kg) par voie orale une heure avant le déclenchement de la diarrhée. Le lopéramide réduit de 50 % l'excrétion fécale d'eau après LPS (40 % pour CU1) et de 60 % après huile de ricin (60 % pour CU1).

### Conclusions

*In vivo,* la souche *Bacillus subtilis* CU1 permet d'améliorer les capacités d'absorption du côlon en condition d'excès de liquide.

Des études complémentaires ont montré que la souche *Bacillus subtilis* CU1 :
- n'a pas d'effet sur les capacités de sécrétion/ absorption de l'intestin grêle dans le modèle huile de ricin et le modèle LPS,
- abolit l'augmentation de perméabilité paracellulaire induite par le LPS au niveau de la paroi du côlon, mais non celle induite par l'huile de ricin,
- réduit le courant de court-circuit induit par l'huile de ricin au niveau de la paroi du côlon, ce qui suggère un renforcement des capacités du côlon à absorber l'eau en situation de diarrhée laxative induite par huile de ricin (et non en situation basale).

La souche *Bacillus subtilis* CU1 et les cellules obtenues par culture de cette souche sont ainsi utiles pour le traitement et/ou la prévention de tout type de diarrhée.

### Résumé de l'Invention

### Souches Probiotiques pour le Traitement et/ou la Prévention de la Diarrhée

La présente invention concerne un procédé de sélection ou d'identification de souches probiotiques capables d'agir sur l'absorption d'eau au niveau du côlon, et leur utilisation comme médicaments dans le traitement et/ou la prévention de la diarrhée. L'invention concerne en particulier la souche de *Bacillus subtilis* CU1 pour son utilisation dans le traitement et/ou la prévention de la diarrhée.
(pas de figure)

## Revendications

1. Procédé de sélection d'une souche probiotique pour le traitement et/ou la prévention de la diarrhée, le procédé comprenant les étapes de :
- mesure de l'effet d'au moins une souche à tester sur l'expression de la protéine CFTR dans un échantillon biologique, et
- sélection d'au moins une souche qui induit une diminution de l'expression de la protéine CFTR,
où l'échantillon biologique est un échantillon d'intestin proximal ou des colonocytes.

2. Procédé de sélection selon la revendication 1, le procédé comprenant en outre les étapes de :
- mesure de l'effet d'au moins une souche à tester sur l'expression de la protéine NHE3 dans un échantillon biologique, et
- sélection d'au moins une souche qui induit une augmentation de l'expression de la protéine NHE3,
où l'échantillon biologique est un échantillon d'intestin proximal ou des colonocytes.

3. Procédé de sélection selon la revendication 1 ou la revendication 2, **caractérisé en ce que** la souche à tester est une souche de levure ou une souche de bactérie.

4. Procédé de sélection selon la revendication 3, **caractérisé en ce que** la souche de bactérie est une souche de *Bacillus,* de préférence une souche de *Bacillus subtilis.*

5. Procédé de sélection selon l'une quelconque des revendications 1 à 4, **caractérisé en ce que** l'expression de la protéine CFTR et/ou de la protéine NHE3 est mesurée par électrophorèse, Western-blot, immunoessai, immunohistochimie, immunocytochimie, spectrométrie de masse, RT-PCR ou Northern-blot.

6. Souche probiotique, ou composition comprenant ladite souche probiotique pour l'utilisation dans le traitement et/ou la prévention de la diarrhée, **caractérisée**(s) en ce que la souche probiotique est la souche de *Bacillus subtilis* CU1 déposée à la CNCM le 25 octobre 2001 sous le numéro I-2745.

7. Souche probiotique, ou composition pour l'utilisation selon la revendication 6, **caractérisée**(s) en ce que la diarrhée est une diarrhée aiguë ou une diarrhée chronique.

8. Souche probiotique, ou composition pour l'utilisation selon la revendication 6 ou la revendication 7, **caractérisée**(s) en ce que la diarrhée est une diarrhée d'origine infectieuse ou une diarrhée d'origine non infectieuse.

## Patentansprüche

1. Verfahren zum Auswählen eines probiotischen Stammes für die Behandlung und/oder Vorbeugung von Diarrhoe, wobei das Verfahren die folgenden Schritte umfasst:
- Messen der Wirkung mindestens eines zu testenden Stamms auf die Expression des Proteins CFTR in einer biologische Probe, und
- Auswählen mindestens eines Stammes, der eine Abnahme der Expression des Proteins CFTR induziert,
wobei die biologische Probe eine proximale Darmprobe oder eine Kolonozytenprobe ist.

2. Verfahren nach Anspruch 1, wobei das Verfahren weiterhin die folgenden Schritte umfasst:
- Messen der Wirkung mindestens eines zu testenden Stamms auf die Expression des Proteins NHE3 in einer biologischen Probe, und
- Auswählen mindestens eines Stamms, der eine Erhöhung der Expression des Proteins NHE3 induziert,
wobei die biologische Probe eine proximale Darmprobe oder eine Kolonozytenprobe ist.

3. Verfahren nach Anspruch 1 oder Anspruch 2, **dadurch gekennzeichnet, dass** der zu testende Stamm ein Hefestamm oder ein Bakterienstamm ist.

4. Verfahren nach Anspruch 3, **dadurch gekennzeichnet, dass** der Bakterienstamm ein *Bacillus* Stamm ist, vorzugsweise ein *Bacillus subtilis* Stamm.

5. Verfahren nach einem der Ansprüche 1 bis 4, **dadurch gekennzeichnet, dass** die Expression des Proteins CFTR und/oder des Proteins NHE3 durch Elektrophorese, Western-Blot, Immunoassay, Immunhistochemie, Immunzytochemie, Massenspektrometrie, RT-PCR oder Northern-Blot gemessen wird.

6. Probiotischer Stamm oder Zusammensetzung welche den probiotischen Stamm umfasst, zur Verwendung für die Behandlung und/oder Vorbeugung von Diarrhoe, **dadurch gekennzeichnet, dass** der probiotische Stamm der *Bacillus subtilis* Stamm CU1 ist, der am 25. Oktober 2001 unter der Nummer I-2745 beim CNCM hinterlegt wurde.

7. Probiotischer Stamm oder Zusammensetzung zur Verwendung nach Anspruch 6, **dadurch gekennzeichnet, dass** die Diarrhoe eine akute Diarrhoe oder eine chronische Diarrhoe ist.

8. Probiotischer Stamm oder Zusammensetzung zur Verwendung nach Anspruch 6 oder Anspruch 7, **dadurch gekennzeichnet, dass** die Diarrhoe eine Diarrhoe infektiösen Ursprungs oder eine Diarrhoe nicht-infektiösen Ursprungs ist.

## Claims

1. Method of selecting a probiotic strain for treating and/or preventing diarrhea, said method comprising the steps of:
- measuring the effect of at least one test strain on expression of the CFTR protein in a biological sample, and
- selecting at least one strain that induces a decrease in expression of the CFTR protein,
wherein the biological sample is a proximal intestine sample or colonocytes.

2. Method of selection according to Claim 1, said method comprising the steps of:
- measuring the effect of at least one test strain on expression of the NHE3 protein in a biological sample, and
- selecting at least one strain that induces an increase in expression of the NHE3 protein,
wherein the biological sample is a proximal intestine sample or colonocytes.

3. Method of selection according to Claim 1 or Claim 2, **characterized in that** the test strain is a yeast strain or a bacterial strain.

4. Method of selection according to Claim 3, **characterized in that** the bacterial strain is a strain of *Bacillus,* preferably a strain of *Bacillus subtilis.*

5. Method of selection according to any one of Claims 1 to 4, **characterized in that** the expression of the CFTR protein and/or of the NHE3 protein is measured by electrophoresis, Western blot, immunoassay, immunohistochemistry, immunocytochemistry, mass spectrometry, RT-PCR or Northern blot.

6. Probiotic strain, or composition comprising said probiotic strain for use in the treatment and/or prevention of diarrhea, **characterized in that** the probiotic strain is the strain of *Bacillus subtilis* CU1 deposited at the CNCM on October 25, 2001 under number 1-2745.

7. Probiotic strain, or composition for use according to Claim 6, **characterized in that** the diarrhea is acute diarrhea or chronic diarrhea.

8. Probiotic strain, or composition for use according to Claim 6 or Claim 7, **characterized in that** the diarrhea is diarrhea of infectious origin or diarrhea of noninfectious origin.
